# EUROPEAN PATENT APPLICATION

(11) **EP 1 095 640 A1**
(43) Date of publication of application: **02.05.2001**
(21) Application number: 00123215.6
(22) Date of filing: 26.10.2000
(51) Int. Cl.: A61F 5/37

(54) **Immobilisation system**

(30) Priority: 29.10.1999 SE 9903903
(71) Applicant: Planet Medical, 5700 Svendborg (DK)
(72) Inventor: Al-Kassim, Adil, 5700 Svendborg (DK)
(74) Representative: Asketorp, Göran

(57) **Abstract**

The present invention refers to an immobilisation system including an immobilisation unit (10) and an immobilisation holder (11). The invention also refers to an immobilisation unit comprising a mouldable sheet (12) and a rigid adjustable frame (13). The immobilisation unit comprises an adjustable frame to which a mouldable sheet of a low temperature melting temperature material is attached. After exposure to an increased temperature the low melting temperature material becomes soft and can be used to make an imprint of a body without cracking. The immobilisation holder being adapted to hold the frame in exact position comprises a mechanism for locking the frame into the holder by a slide in, pressure, or snap mechanism to obtain a tight connection between the frame and the holder and thereby obtain a perfect immobilisation of the body.

## Description

### FIELD OF INVENTION

The present invention refers to an immobilisation system including an immobilisation unit comprising a mouldable sheet and an adjustable frame and an immobilisation holder for use in immobilisation.

### BACKGROUND

Low temperature thermoplastic materials are currently used in immobilisation applications in medical therapy treatments, such as in making orthopedic splints and in the immobilisation of various body parts of patients or animals undergoing medical therapy treatments such as radiation. Such thermoplastic materials are usually formed as sheets, strips or rods. They may or may not be perforated. Such thermoplastic materials when heated to approximately 45° C become soft and further heating to temperature rising from 60° C to 80° C become completely soft. In practical use these materials may be placed in an oven or a liquid solution such as a water solution preheated to the softening temperature of the thermoplastic material.

As the material is heated it softens and becomes pliable /formable. The material is then removed from heating, placed round the body required to be immobilised. As the material cools down to room temperature, it solidifies retaining its formed shape.

The need of immobilisation arises from the requirement to irradiate specific, well-defined sections/volumes of the human body while minimising exposure to the surrounding human body. These sections are determined from a detailed medical investigation such as by means of x-ray, Computerised Tomography (CAT) or other imaging techniques. As a result of the investigation a treatment is planned normally with the aid of a sophisticated treatment-planning computer. In treatment planning, factors such as dose level, duration, volume to be irradiated, number of treatments and angle/angles of irradiation are determined. Further the patient normally will be treated several times in which the same position is highly important to maintain. To be able to assure that the radiation is delivered exactly where it is needed it is highly important that the patient is immobilised during the treatment and further immobilised at the same position from time to time of treatment. Although the treatments are planned /calculated to a high position and although it is possible to re-position the patient very accurately, patients movement (including involuntary movements such as those related to breathing, swaying, etc.) are present and must be accounted for. This emphasises the need for an immobilisation system to be able to fixate the patient in exactly the same position from treatment to treatment.

Various immobilisation techniques have been devised and are today in use. The main ones are; vacuum forming of high temperature thermoplastics on a gypsum or a two component polyurethane replicas of the patient (VFHT) or direct forming a low temperature thermoplastics on the patient (DLT).

One problem with the DLT technique mentioned above is that due to the solid frame construction the amount of thermoplastic material included in the sheet is substantially predetermined. Thus, when the frame construction is to be used for treatment of a large body the thermoplastic sheet is often stretched up to a point where the sheet becomes thin and easy to break. Further, the moulding should take place as fast as possible due to that the sheet start to become rigid when the temperature decrease and therefore it is highly important to fixate the immobilisation means as fast as possible to the immobilisation holder. Commonly used techniques use a subset of screws to fixate the immobilisation means to the holder. This technique could result in bending of the frame during the immobilisation and later on inaccurate repeatable fixation of the body.

### OUTLINE OF THE INVENTION

An object of the present invention is to provide an immobilisation system and unit for use in immobilisation of a body which overcomes the above mentioned drawbacks.

The present invention refers to an immobilisation system, for use in immobilisation of a body including an immobilisation unit and an immobilisation holder, wherein said unit comprises a mouldable sheet and a rigid, adjustable frame for use in immobilisation, said frame co-operating with attachment fittings in or on the immobilisation holder to which the immobilisation unit is to be attached.

The present invention also refers to an immobilisation unit comprising a mouldable sheet and an adjustable frame.

Thus, the present invention refers to an immobilisation system including an immobilisation unit and an immobilisation holder. The invention also refers to an immobilisation unit comprising a mouldable sheet and a rigid adjustable frame. The immobilisation unit comprises an adjustable frame to which a mouldable sheet of a low temperature melting material is attached. After exposure to an increased temperature the low melting temperature material becomes soft and can be used to make an imprint of a body without cracking. The immobilisation holder being adapted to hold the frame in exact position comprises a mechanism for locking the frame into the holder by e.g. a slide in, pressure, or snap mechanism to obtain a tight connection between the frame and the holder and thereby obtain a perfect immobilisation of the body.

The immobilisation unit comprises a mouldable sheet and a rigid, adjustable frame with attachment fittings adapted to co-operate with attachments in or on the immobilisation holder. Preferably the present invention is used for immobilisation of a body, such as human or animal. More preferably the present invention is used for immobilisation of a body in medical therapy, such as in radiotherapy or other medical therapies. The mouldable sheet is preferably made of a low melting temperature material, such as a low temperature thermoplastic material. The adjustable frame is made of a rigid material.

A benefit of the present immobilisation system and unit is that the frame, being adjustable, is capable of assembling a larger thermoplastic sheet than a solid frame. Thus, the present adjustable frame comprises more material making it possible to stretch the sheet further without breaking the material.

According to another embodiment the present immobilisation system and unit are characterised in that the frame can be made in one integral piece manufactured in its stretched condition so that the user has to compress it in the moulding temperature to make it fit in the immobilisation holder.

According to another embodiment the present immobilisation system and unit are characterised in that the frame can be made in one integral piece manufactured in its compressed condition so that the user has to stretch it in the moulding temperature to make it fit the immobilisation holder.

According to another embodiment the present immobilisation system and unit are characterised in that the frame can be made adjustable through a telescopic mechanism either in a 2,3 or more pieces.

According to another embodiment the present immobilisation system and unit are characterised in that the immobilisation holder comprise a fast fitting lock for the frame comprising three locking devices for the frame.

According to a preferred embodiment the frame is provided with two holes for locking the ends of the frame wings to the holder and a snap fastener mechanism for locking the top to the holder.

According to another embodiment the present immobilisation system and unit are characterised in that the frame wings comprise a bulged area for optimal attachment to the immobilisation holder.

According to another embodiment the present immobilisation system and unit are characterised in which the sheet after use can be replaced with a new sheet into the frame, i.e. the sheet may be disposable. Thus, the sheet may be replaceably or permanently attached to the frame.

### SHORT DESCRIPTION OF DRAWINGS

- Fig. 1: is a top view showing, in accordance with the present invention, an immobilisation system including an immobilisation unit and an immobilisation holder.
- Fig. 2: is side elevation view of the system in Fig. 1.
- Fig. 3: is a plan view from below of an empty frame in a closed position included in the immobilisation unit.
- Fig. 4: is a plan view from below of an alternative embodiment of an empty frame in a closed position included in the immobilisation unit.
- Fig. 5: is a plan view from below of the frame in Fig. 3 in an open position supporting a mouldable sheet before exposure to an increased temperature.
- Fig. 6: is a plan view from above of an empty immobilisation holder.
- Fig. 7A: is side elevation view of a first embodiment of a frame having a curved section.
- Fig. 7B: is side elevation view of a second embodiment of a frame having a curved section.

### DETAILED DESCRIPTION OF THE INVENTION

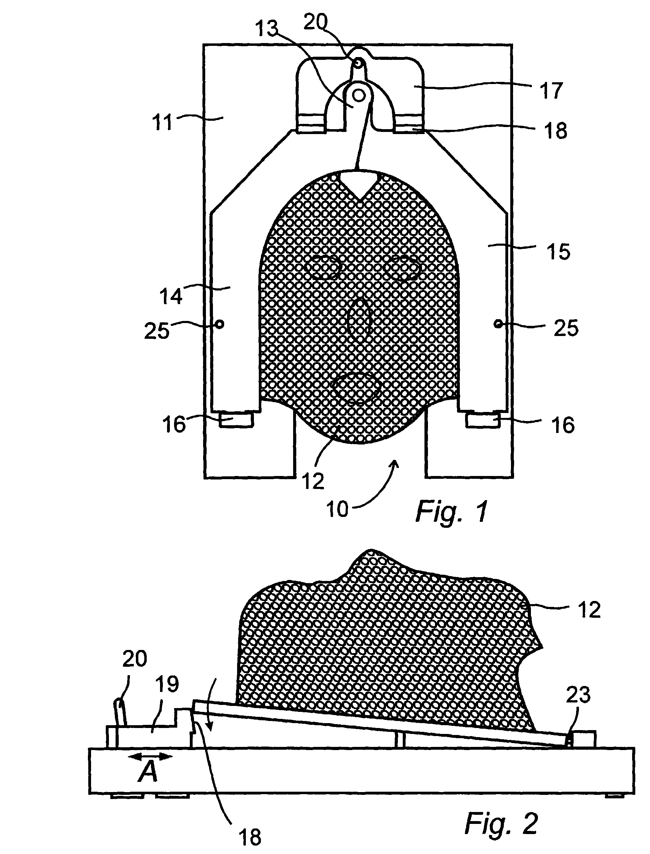

In the embodiment of the invention shown in Fig. 1 there is included a complete immobilisation system including an immobilisation unit 10 and an immobilisation holder 11. The immobilisation unit 10 includes a rigid, adjustable frame 13 holding a thermoplastic sheet 12 that has been formed over the face of a patient. The frame 13 includes a first curved wing 14 and a second curved wing 15 forming together a U-shape.

The frame 13 is attached to the holder 11 by attachment means 16 and a locking means 17. In the embodiment shown in Fig. 1 and Fig. 2 the attachment means 16 comprises two stop pieces, each one being formed with a slot to receive a protruding section 23 of the wings 14, 15. The locking means comprises a U-shaped displaceable block 19 with two inclined shoulder sections 18. The displaceable block 19 can be displaced by moving a snap peg 20 to release the immobilisation unit 10 from the holder.

Fig. 2 shows a position of the immobilisation unit 10 where it is pressed down over the inclined shoulder sections 18 to be locked in a space below the shoulder sections. In Fig. 2 the adjustable frame 13, containing a hinge region attached to the low melting temperature thermoplastic sheet 12, has been treated in a water solution to an increased temperature of 60°C to 80°C whereby the low temperature thermoplastic sheet becomes soft and mouldable. The frame remains rigid. The preheated immobilisation unit has then been transferred and pressed onto a body, in this case a face of a human body by locking the wings of the frame into two pockets in the immobilisation holder and pressing down the middle part of the frame containing the hinge over the face against the upper part of the face. Thereby the moulding sheet will be pressed and formed around the body of the face. The hinge will further be pressed down against the immobilisation holder into a snap grip and locked.

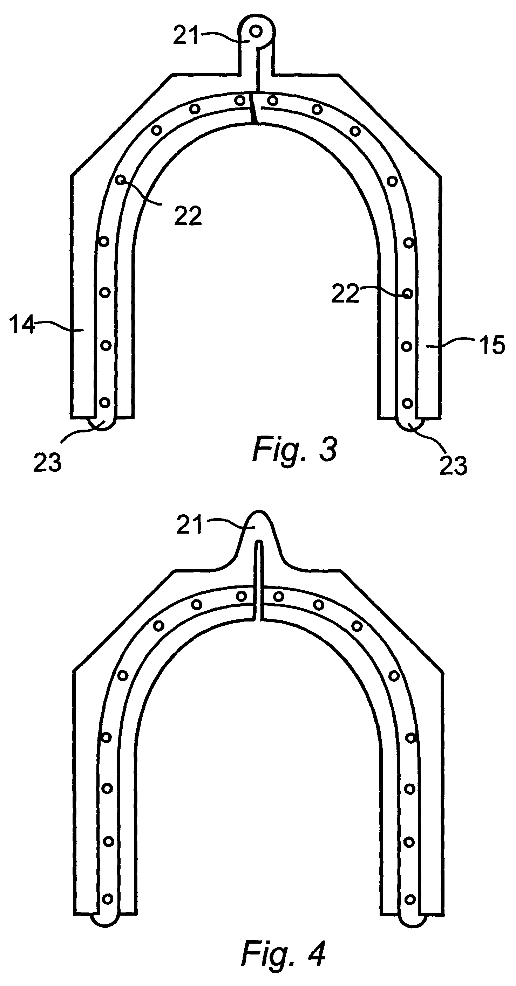

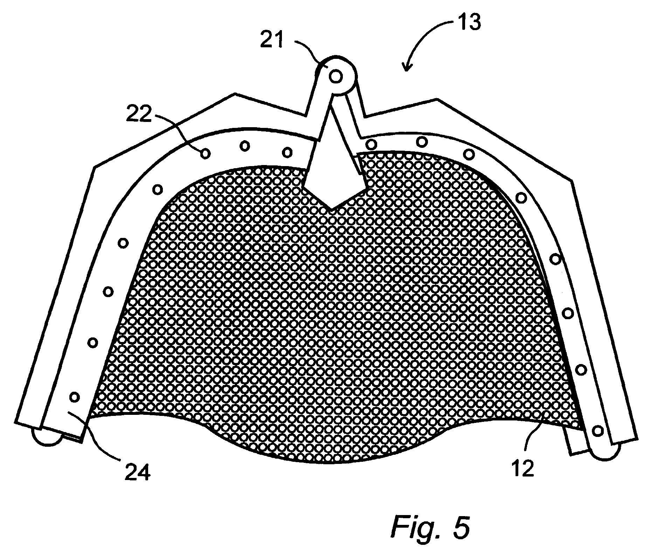

Fig. 3 shows a first embodiment of the frame 13 comprising two wings 14, 15 that are connected in a hinged section 21. The hinged section will allow the frame to be opened at least to a position as shown in Fig. 5. Along a curved inner part of each of the wings there are provided a plurality of pegs 22 that will facilitate the attachment of the mouldable sheet to the frame. At a free end of each of the wings a curved section 23 protrudes.

According to the embodiment shown in Fig. 4 the present immobilisation system and unit are characterised in that the frame 13 can be made in one integral piece including a hinge region or section 21 of appropriate raggedness and collapsing resistance. It can be manufactured in its compressed condition so that the user has to stretch it in the moulding temperature to make it fit the immobilisation holder. It may also be manufactured in its stretched condition so that the user has to compress it in the moulding temperature to make it fit in the immobilisation holder.

The use of such thermoplastic materials is not restricted to the above applications and can be used for other applications where fixation or immobilisation of a body is required. The expression "body" is herein used in a wide sense, including bodies of any materials.

In Fig. 5 the sheet 12 has been mounted in the frame 13. An adhesive can be applied to the frame wings to enhance the connection. On each of the wings there are attached a locking rim 24, only one being attached in Fig. 5. Preferably both the locking rims 24 and the wings are provided with elongated ridges that will co-operate and lock tightly the sheet 12 there between. Preferably the pegs 22 securely fasten the locking rims 24.

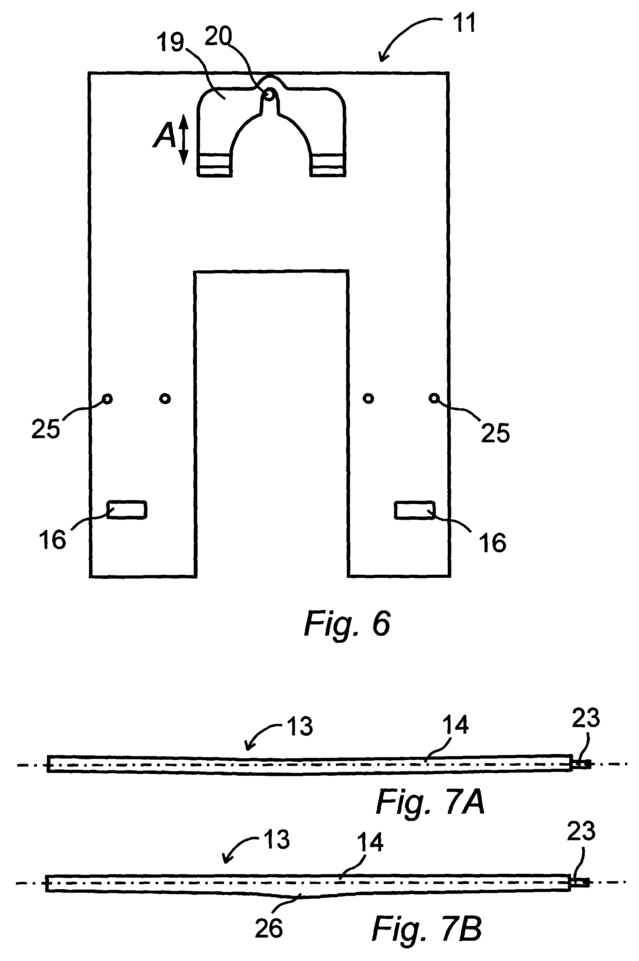

Fig. 6 shows an empty holder 11. A cut out mid section gives the holder a U-shape. Each leg of the holder is provided with attachment means 16 and attachment pegs 25 for the attachment of an immobilisation unit. An arrow A shows how the block 19 can be moved back and forth during mounting and demounting of the immobilisation unit 10 in the holder 11.

Fig. 7A and Fig. 7B show two embodiments of the frame 13. In the embodiment shown in Fig. 7A the frame wings 14, 15 comprise an upwardly curved area for an optimal attachment to the immobilisation holder 11. The frame wings can be manufactured with upwards curvatures at the centre of each wing to provide pressure in the centre when the frame is fitted into the locking mechanism. In the embodiment shown in Fig. 7B the frame wings comprise a thicker central section than at the ends of each wing.

According to one embodiment the present immobilisation system and unit are characterised in that the adjustable frame is made of a material which does not deform under working temperature and that the mouldable sheet is made of a low melting temperature thermoplastic material. The frame is made of a rigid material, such as a high temperature melting material. The frame may also be made of any other suitable rigid material such as wood and steel.

According to another embodiment the present immobilisation system and unit are characterised in that the frame 13 and the sheet 12 are attached to each other by methods comprising chemical, UV or electron beam pasting, screwing or by any other method. In any embodiment the frame may engage essentially the whole circumference section of the mouldable sheet.

According to another embodiment the present immobilisation system is characterised in that the immobilisation unit is exposed to an increased temperature prior to moulding.

According to another embodiment the present immobilisation system is characterised in that immobilisation unit is attached to an immobilisation holder after moulding.

The immobilisation unit of present invention comprises a sheet of mouldable material, preferably a low melting temperature thermoplastic material, that after moulding becomes rigid and an adjustable frame of a rigid material. Preferably the rigid material is selected from the group of high melting temperature thermoplastic material or any other thermo resistant material. The frame and the sheet may be attached to each other by melting, by means of adhesives, screws, clamps or in any other way. Any known chemical/UV or electron beam adhesives may be used. The immobilisation unit is exposed to an increase temperature above 45°C preferably between 60°C to 80°C such that the low thermoplastic material become soft and the high thermoplastic material remain rigid so that it can be used to attach the immobilisation means into the holder. The perforation percentage and thus the hole size may vary depending on application and stability requirements.

The frame according to the present invention is preferably prepared and presented in an arch-form or U-form. In one embodiment the frame has an arch form, with two wings and being capable of assembling a larger thermoplastic sheet initially compared to the final after moulding. Further the frame can be made i) in one integral piece manufactured in its stretched condition so that the user has to compress it during the moulding procedure to make it fit into the immobilisation holder, ii) in one integral piece manufactured in its compressed condition so that the user has to stretch it during the moulding procedure to make it fit into the immobilisation holder, iii) in two pieces connected at one end with a hinge having appropriate raggedness and collapsing resistance or iv) adjustable through a telescopic mechanism either in two or three or more pieces. Preferably the frame is manufactured as one integral piece having a collapsible hinge in the middle. During the moulding procedure the hinge collapses. Further the frame can be in a plane form or preferable in a form in which the wings are made in a curved or bulged form for optimal attachment to the immobilisation holder.

After the exposure to an increased temperature the immobilisation unit is placed onto the body to be moulded and further immobilised into the holder by an attachment mechanism.

The attachment mechanism, which locks the immobilisation unit and holder firmly together, may be construed in various ways. It may e.g. be in the form of screws, clamps or other locking mechanisms. Preferably the lock unit should comprise a simple slide, pressure, snap mechanism, such as two holding pockets into which the frame wings fit by sliding and further a third snap grip which permanently fixate the immobilisation unit when the frame is pressed therein. Further, the immobilisation means can be fixated into the holder by the slide, pressure, snap mechanism and released by a fast and simple release mechanism such as a snap peg. The snap peg located in the snap grip release the immobilisation unit through moving the snap peg vertically away from the immobilisation unit, which also moves the snap grip vertically away from the immobilisation unit and thereby releases it.

## Claims

1. Immobilisation system for use in immobilisation of a body including an immobilisation unit and an immobilisation holder, wherein said unit comprises a mouldable sheet and a rigid, adjustable frame for use in immobilisation, said frame co-operating with attachment fittings in or on the immobilisation holder to which the immobilisation unit is to be attached.

2. Immobilisation system according to claim 1, characterised in that the adjustable frame is made of a material which does not deform under working temperature and that the mouldable sheet is made of a low melting temperature thermoplastic material .

3. Immobilisation system according to claim 1 or 2, characterised in that the frame is capable of assembling a larger thermoplastic sheet initially than the final size after moulding.

4. Immobilisation system according to claims 1 ―3, characterised in that the frame has an arch-form, one part (the inner part) attached to the mouldable sheet and the other part to the immobilisation holder.

5. Immobilisation system according to claims 1-4, characterised in that the frame and the sheet are attached to each other by methods comprising chemical, UV, electron beam pasting, screwing or any other way.

6. Immobilisation system according to claims 1-5, characterised in that the frame engages essentially the whole circumference section of the mouldable sheet.

7. Immobilisation system according to claims 1-6, characterised in that the immobilisation unit is exposed to an increased temperature prior moulding.

8. Immobilisation system according to claims 1-7, characterised in that the immobilisation unit is attached into an immobilisation holder after moulding.

9. Immobilisation system according to claims 1-8, characterised in that the frame is made in one integral piece manufactured in its stretched condition so that the user compresses it in the moulding temperature to make it fir in the immobilisation holder.

10. Immobilisation system according to claims 1-8, characterised in that the frame is made in one integral piece manufactured in its compressed condition so that the user has to stretch it in the moulding temperature to make it fit the immobilisation holder.

11. Immobilisation system according to claims 1-8, characterised in that the frame is made of 2 pieces (wings) connected at one end with a hinge region of appropriate raggedness and collapsing resistance.

12. Immobilisation system according to claims 1-8, characterised in that the frame is made adjustable through a telescopic mechanism either in a 2,3 or more pieces.

13. Immobilisation system according to claims 1-12, characterised in that the immobilisation holder comprises a fast fitting lock for the frame comprising three locking devices for the frame.

14. Immobilisation system according to claims 1-13, characterised in that the frame wings comprise an upwardly curved area for optimal attachment to the immobilisation holder.

15. Immobilisation system according to claims 1-13, characterised in that the frame wings comprise a bulged area for optimal attachment to the immobilisation holder.

16. Immobilisation system, characterised in that the sheet after use can be replaced with a new sheet into the frame.

17. Immobilisation unit having a frame and a sheet, characterised in that the frame is adjustable.

18. Immobilisation unit according to claim 17, characterised in that the sheet is made of a low melting temperature thermoplastic material.

19. Immobilisation unit according to claim 17, characterised in that the adjustable frame is made of a material which does not deform under working temperature and that the mouldable sheet is made of a low melting temperature thermoplastic material .

20. Immobilisation unit according to claims 17-19, characterised in that the frame is capable of assembling a larger thermoplastic sheet initially than the final size after moulding.

21. Immobilisation unit according to claims 17-20, characterised in that the frame has an arch-form, one part (the inner part) attached to the mouldable sheet and the other part to the immobilisation holder.

22. Immobilisation unit according to claims 17-21, characterised in that the frame and the sheet are attached to each other by methods comprising chemical, UV, electron beam pasting, screwing or any other way.

23. Immobilisation unit according to claims 17-22, characterised in that the frame engages essentially the whole circumference section of the mouldable sheet.

24. Immobilisation unit according to claims 17-23, characterised in that the frame is made in one integral piece manufactured in its stretched condition so that the user compresses it in the moulding temperature to make it fit in the immobilisation holder.

25. Immobilisation unit according to claims 17-23, characterised in that the frame is made in one integral piece manufactured in its compressed condition so that the user has to stretch it in the moulding temperature to make it fit the immobilisation holder.

26. Immobilisation unit according to claims 17-23, characterised in that the frame is made of 2 pieces (wings) connected at one end with a hinge region of appropriate raggedness and collapsing resistance.

27. Immobilisation unit according to claims 17-23, characterised in that the frame is made adjustable through a telescopic mechanism either in a 2,3 or more pieces.

28. Immobilisation unit according to claim 26, characterised in that the frame wings comprise an upwardly curved area for optimal attachment to the immobilisation holder.

29. Immobilisation unit according to claim 26, characterised in that the frame wings comprise a bulged area for optimal attachment to the immobilisation holder.

30. Immobilisation unit according to claims 17-29 , characterised in that the sheet after use can be replaced with a new sheet into the frame.
